(19) Europäisches Patentamt
European Patent Office
Office européen des brevets

(11) Veröffentlichungsnummer: **0 421 227 B1**

(12) **EUROPÄISCHE PATENTSCHRIFT**

(45) Veröffentlichungstag der Patentschrift: **15.06.94**

(51) Int. Cl.5: **C07D 249/08**, C07D 409/12, C07D 401/12, C07D 413/12, A01N 43/653

(21) Anmeldenummer: **90118281.6**

(22) Anmeldetag: **24.09.90**

# (54) Derivate des 1-Hydroxy-1,2,4-triazols und diese enthaltende Fungizide und wachstumsregulierende Mittel.

(30) Priorität: **30.09.89 DE 3932752**

(43) Veröffentlichungstag der Anmeldung:
**10.04.91 Patentblatt 91/15**

(45) Bekanntmachung des Hinweises auf die Patenterteilung:
**15.06.94 Patentblatt 94/24**

(84) Benannte Vertragsstaaten:
**AT BE CH DE DK ES FR GB GR IT LI NL SE**

(56) Entgegenhaltungen:
**EP-A- 0 101 288**
**EP-A- 0 384 365**

**SYNTHESIS, Nr. 10, Oktober 1989, Seiten 773-775; G. LAUS et al.: "1-Hydroxyimidazole derivatives III. Synthesis of 1-alkyloxy-, 1-arylalkyloxy-, and 1-phenoxy-1H-imidazoles"**

(73) Patentinhaber: **BASF Aktiengesellschaft**
**Carl-Bosch-Strasse 38**
**D-67063 Ludwigshafen(DE)**

(72) Erfinder: **Sauter, Hubert, Dr.**
**Neckarpromenade 20**
**D-6800 Mannheim 1(DE)**
Erfinder: **Zierke, Thomas, Dr.**
**Bahnhofstrasse 10**
**D-6737 Boehl-Iggelheim(DE)**
Erfinder: **Reuther, Wolfgang, Dr.**
**Am Pferchelhang 16**
**D-6900 Heidelberg(DE)**
Erfinder: **Baus, Ulf, Dr.**
**Im Hallgarten 6**
**D-6915 Dossenheim(DE)**
Erfinder: **Lorenz, Gisela, Dr.**
**Erlenweg 13**
**D-6730 Neustadt(DE)**
Erfinder: **Ammermann, Eberhard, Dr.**
**Sachsenstrasse 3**
**D-6700 Ludwigshafen(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 421 227 B1

Rank Xerox (UK) Business Services
(3.10/3.09/3.3.3)

## Beschreibung

Die vorliegende Erfindung betrifft neue Derivate des 1-Hydroxy-1,2,4-triazols, deren Herstellung, deren Verwendung als Fungizide und Pflanzenwachstumsregulatoren, fungizide und pflanzenwachstumsregulierende Mittel, die die neuen Wirkstoffe enthalten sowie Verfahren zur Bekämpfung von Pilzen und zur Regulierung des Pflanzenwachstums mit diesen Wirkstoffen.

Aus EP 165777 sind Azolderivate bekannt, wie z. B. 1-Bis (4-fluorphenyl)methyl-(1H-1,2,4)triazol der Formel

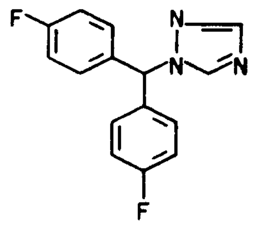

bekannt, die als Aromatase-Inhibitoren wirken. Ihre fungizide Wirkung ist jedoch nicht ausreichend.

Es wurden nun neue Verbindungen der Formel I gefunden

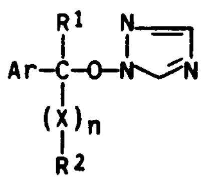

I

in welcher

- Ar für gegebenenfalls ein- oder mehrfach durch Halogen, $C_1$-$C_4$-Alkoxy, $C_1$-$C_4$-Alkyl, Trifluormethyl, $C_1$-$C_4$-Alkoximino, gegebenenfalls durch Halogen oder Trifluormethyl substituiertes Phenyl oder Phenoxy substituiertes Phenyl, gegebenenfalls durch die gleichen Reste substituiertes Pyridyl, gegebenenfalls durch die gleichen Reste substituiertes Thienyl oder gegebenenfalls durch die gleichen Reste substituiertes Naphthyl steht,
- $R^1$ für Wasserstoff steht oder für den Fall, daß n = 0 ist, zusätzlich für CN steht,
- $R^2$ für gegebenenfalls ein- oder mehrfach durch Halogen, $C_1$-$C_4$-Alkyl, $C_1$-$C_4$-Alkoximino $C_1$-$C_4$-Alkoxy oder Trifluormethyl substituiertes Aryl, gegebenenfalls durch die gleichen Reste substituiertes Heteroaryl steht oder für gegebenenfalls durch $C_1$-$C_4$-Alkyl substituiertes $C_3$-$C_8$-Cycloalkyl steht oder für geradkettiges oder verzweigtes, gegebenenfalls ein- oder mehrfach durch Halogen $C_3$-$C_6$-Cycloalkyl, Aryl, Aryloxy substituiertes $C_1$-$C_6$-Alkyl, gegebenenfalls durch die gleichen Reste substituiertes $C_2$-$C_6$-Alkenyl oder gegebenenfalls durch die gleichen Reste substituiertes $C_2$-$C_6$-Alkinyl steht, und
- X für $CH_2$, O oder S steht und
- n 0 oder 1 bedeutet, sowie deren pflanzenverträgliche Säureadditionssalze und Metallkomplexe, die eine sehr hohe fungitoxische Wirkung bei ausgezeichneter pflanzenverträglichkeit aufweisen. Weiterhin zeigen sie auch bei niedrigen Aufwandmengen starke wachstumsregulierende Wirkungen bei sehr guter Pflanzenverträglichkeit.

Die neuen Verbindungen der Formel I enthalten im allgemeinen chirale Zentren. Sie werden im allgemeinen als Racemate oder gegebenenfalls als Diastereomerengemische erhalten. Diastereomerenreine Verbindungen lassen sich bei einigen der neuen Verbindungen durch Destillation, Säulenchromatographie oder aufgrund von Löslichkeitsunterschieden isolieren. Enantiomerenreine Verbindungen lassen sich z. B. durch Racematspaltung mit einem chiralen Hilfsreagenz nach bekannten Methoden beispielsweise über diastereomere Salze erhalten. Für die Anwendung der neuen Verbindungen als Fungizide und Wachstumsregulatoren sind sowohl die Diastereomeren bzw. die Enantiomeren als auch die bei der Synthese anfallenden Stereoisomerengemische geeignet. Sie alle werden von der Erfindung umfaßt.

Ar bedeutet beispielsweise Phenyl, Halogenphenyl, 4-Fluorphenyl, 4-Chlorphenyl, 4-Bromphenyl, 4-Trifluormethylphenyl, $C_1$-$C_4$-Alkylphenyl, 4-Methylphenyl, 4-tert.-Butylphenyl, $C_1$-$C_4$-Alkoxyphenyl, 4-Me-

thoxyphenyl, $C_1$-$C_4$-Alkoxiiminophenyl, 4-Methoximinophenyl, 2,4-Dichlorphenyl, 3,4-Dichlorphenyl, 2,6-Dichlorphenyl, 3-Trifluormethylphenyl, 3-Fluorphenyl, 2-Fluorphenyl, 2-Chlorphenyl, 2-Bromphenyl, 2-Methylphenyl, 4-Fluor-2-Chlorphenyl, 4-Chlor-2-Fluorphenyl, 4-Ethoximinophenyl, 2-Methoxyphenyl, 2-Pyridyl, 3-Pyridyl, 4-Pyridyl, 1-Naphthyl, 2-Naphthyl, 2-Thienyl, 3-Thienyl, 4-n-Butoxyphenyl, 3,5-Dimethoxyphenyl, 2-Methoxy-4-methylphenyl, 3-Chlorphenyl, 3,5-Dichlorphenyl, 2-Methyl-4-Chlorphenyl.

$R^2$ bedeutet beispielsweise $C_1$-$C_6$-Alkyl, insbesondere $C_1$-$C_4$-Alkyl, Methyl, Ethyl, n-Propyl, iso-Propyl, n-Butyl, Isobutyl, But-2-yl, tert.-Butyl, Pentyl, Pent-2-yl, neo-Pentyl, Hexyl, Hex-2-yl, Benzyl, Halogenbenzyl, 4-Chlorbenzyl, 2-Chlorbenzyl, 4-Fluorbenzyl, 2,4-Dichlorbenzyl, 2-Fluorbenzyl, $C_1$-$C_4$-Alkylbenzyl, 2-Methylbenzyl, 2-Phenylethyl, 2-(4-Chlorphenyl)-ethyl, 2-(4-Fluorphenyl)-ethyl, 2-(2-Methylphenyl)-ethyl, 2-(2,4-Dichlorphenyl)-ethyl, 2-(4-Methylphenyl)-ethyl, 2-(2-Chlorphenyl)-ethyl, 2-(2-Chlor-4-Fluorphenyl)-ethyl, 2-(4-tert.-Butylphenyl)-ethyl, $C_3$-$C_8$-Cycloalkyl, Cyclopropyl, 1-Methylcyclopropyl, Cyclobutyl, Cyclopentyl, Cyclohexyl, 4-Methylcyclohexyl, Cycloheptyl, Cyclooctyl, Phenyl, Halogenphenyl, 4-Fluorphenyl, 4-Chlorphenyl, 4-Bromphenyl, 4-Trifluormethylphenyl, $C_1$-$C_4$-Alkoxyphenyl, 4-Methoxyiminophenyl, 2,4-Dichlorphenyl, 3,4-Dichlorphenyl, 2,6-Dichlorphenyl, 3-Trifluormethylphenyl, 3-Fluorphenyl, 2-Fluorphenyl, 2-Chlorphenyl, 3-Chlorphenyl, 3,5-Dichlorphenyl, 2-Methyl-4-chlorophenyl, 4-n-Butoxyphenyl, 3,5-Dimethoxyphenyl, 2-Methoxy-4-methylphenyl, 2-Bromphenyl, 2-Methylphenyl, 4-Fluor-2-Chlorphenyl, 4-Chlor-2-Fluorphenyl, 4-Ethoximinophenyl, 2-Methoxyphenyl, Phenoxyethyl, 2-Fluorphenoxyethyl, 4-Fluorphenoxybutyl, 4-Fluorphenoxypropyl, 4-Chlorphenoxyethyl, 2-Fluorphenoxybutyl, 2-Fluorphenoxyethyl, 2,4-Dichlorphenoxyethyl, 4-Fluorphenoxymethyl, 4-Chlorphenoxymethyl, 2,4-Dichlorphenoxymethyl, 2-Chlorphenoxymethyl, 2,6-Dichlorphenoxymethyl, 4-Methoxyphenoxymethyl, 4-tert.-Butylphenoxymethyl, 2-Methylphenoxymethyl, Pyridyl, 2-Pyridyl, 3-Pyridyl, 4-Pyridyl, Triazolyl, 1,2,4-Triazol-1-yl, Imidazol-1-yl, Isoxazolyl, 3-Alkylisoxazol-5-yl, 3-Isopropylisoxazol-5-yl, Thiazolyl, Pyrimidyl, Pyrrolyl, Pyrazolyl, Naphthyl, 1-Naphthyl, 2-Naphthyl, Thienyl, 2-Thienyl, 3-Thienyl, 2-Chlorthien-3-yl, 3-Bromthien-2-yl, $C_2$-$C_4$-Alkenyl, Ethenyl, Halogen-$C_2$-$C_4$-alkenyl, trans-2-Chlorethenyl, 2,2-Dichlorethenyl, cis-2-Chlorethenyl, Allyl, 2-Butenyl.

Geeignete pflanzenverträgliche Säureadditionssalze sind beispielsweise die Bromide, Sulfate, Nitrate, Phosphate, Oxalate oder Dodecylbenzolsulfonate. Die Wirksamkeit der Salze geht auf das Kation zurück, so daß die Wahl des Anions im allgemeinen beliebig ist.

Geeignete pflanzenverträgliche Metallkomplexe sind Verbindungen der Formel VI

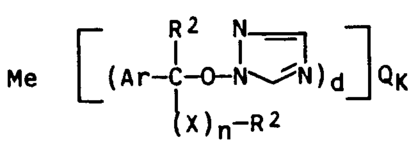

VI

in der Ar, $R^1$, $R^2$, X und n die oben angegebene Bedeutung haben und Me ein Metall, z. B. Cu, Zn, Sn, Mn, Fe, Co oder Ni bedeutet, Q für das Anion einer anorg. Säure, z. B. HCl, $H_2SO_4$, $H_3PO_4$ steht und d und k 1, 2, 3 oder 4 bedeuten. Sie werden z.B. hergestellt durch Umsetzung der Verbindungen der Formel I mit einem Metallsalz z.B. Kupfersulfat, Zinksulfat, Zinnchlorid, Mangansulfat.

Weiterhin wurde gefunden, daß man Azolverbindungen der Formel V erhält, wenn man Verbindungen der Formel II

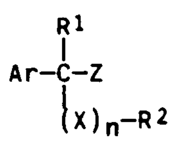

II

in welcher
Ar, $R^1$, $R^2$, X und n die oben angegebene Bedeutung haben und Z für eine nucleofuge Abgangsgruppe, wie Chlor, Brom, Methansulfonat, Benzolsulfonat 4-Tolylsulfonat steht,

a) mit einem Alkalisalz der Formel III

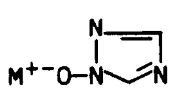

III

in der M für Natrium oder Kalium steht, ggf. in Gegenwart eines Verdünnungsmittels oder

b) mit 1-Hydroxy-1,2,4-triazol der Formel IV

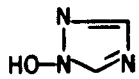

IV

in Gegenwart einer Base der Formel V $M^+B^-$ in welcher M für Natrium oder Kalium steht und B die Bedeutung Hydroxid, Alkoxid, Carbonat oder Hydrid hat, ggf. in Gegenwart eines Verdünnungsmittels umsetzt und die so erhaltenen N-Hydroxytriazolderivate ggf. mit Metallsalzen in ihre pflanzenverträglichen Metallkomplexe überführt und ggf. in Gegenwart eines Verdünnungsmittels umsetzt.

Dabei ist es zweckmäßig, die Verbindungen der Formel II ohne Verdünnungsmittel oder in Gegenwart eines Verdünnungsmittels mit etwa 0,5-2 Äquivalenten eines Alkalisalzes der Formel III oder mit etwa 0,5-4 Äquivalenten des N-Hydroxytriazols der Formel IV unter Zusatz von 0,5-4 Äquivalenten Basen bei Temperaturen von etwa 0 bis 200 °C, vorzugsweise +20 bis +160 °C in homogener oder inhomogener Phase umzusetzen.

Als Verdünnungsmittel können z. B. Methanol, Ethanol, Isopropanol, n-Butanol, tert.-Butanol, Diethylether, Methyl-tert.-butylether, Tetrahydrofuran, Dioxan, Aceton, Acetonitril, Dimethylformamid, Dimethylsulfoxid, Chloroform, Methylenchlorid oder Toluol verwendet werden. Als Basen können z.B. Natrium- oder Kalium-hydroxid, -hydrid, -methanolat, -ethanolat, -isopropanolat, -butanolat, -tert.-Butanolat oder auch -carbonat verwendet werden.

Die Herstellung des 1-Hydroxy-1,2,4-Triazols wird in Beispiel 1 beschrieben.

Die Verbindungen der Formel II sind z. T. aus der Literatur bekannt oder können nach literaturbekannten Verfahren hergestellt werden.

A) So erhält man z. B. α-Chlorsulfide der Formel II (X = S, Z = Cl, $R^1$ = H)

a) durch Chlorierung von Sulfiden mit N-Chlorsuccinimid (siehe z. B. B. L. Tuleen und T. B. Stephens J. Org. Chem., 34, 31 [1969] ) z.B. nach dem Schema

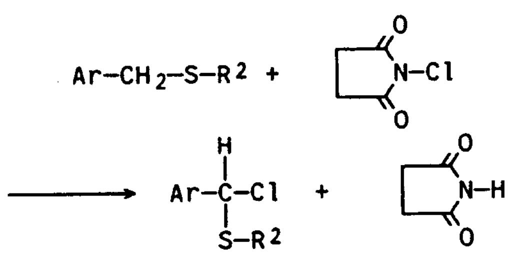

oder

b) durch Umsetzung von Aldehyden mit Thiolen in Gegenwart von Chlorwasserstoff (siehe z. B. H. Böhmer, H. Fischer und R. Frank Liebigs Ann. Chem. 563, 54 1949 ) nach dem Schema

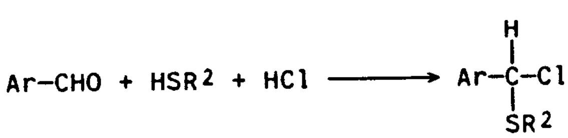

B) α-Chlorether der Formel II (x = O, n = 1, Z = Cl, $R^1$ = H) erhält man z. B.

a) durch Umsetzung von Acetalen mit Acetylchlorid (siehe z. B. D. M. Bailey J. Med. Chem. 17, 702 1974 ) nach dem Schema

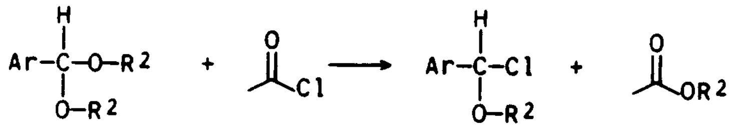

oder

b) durch Chlorierung von Ethern (siehe z. B. E. Vilsmeier Liebigs Ann. Chem. 728, 12 [1969] nach dem Schema

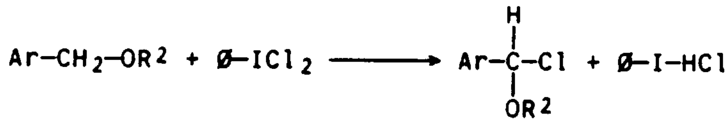

C) Verbindungen der Formel II, in denen X für $CH_2$, $R^1$ für H steht und n = 0 oder 1 sein kann, erhält man entweder

a) durch Umsetzung von Aldehyden der Formel VII

Ar-CHO VII

mit 0,8 bis 1,2 Äquivalenten einer Grignardverbindung der Formel VIII

$R^2$-$(X)_n$ Mg Hal VIII

oder

b) durch Umsetzung von Aldehyden der Formel IX

$R^2$-$(X)_n$-CHO IX

mit 0,8 bis 1,2 Äquivalenten einer Grignardverbindung der Formel X

Ar-Mg Hal X

oder

c) durch Reduktion von Ketonen der Formel XI

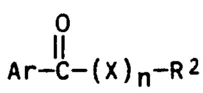

XI

z. B. $C_1$) durch Einwirkung von komplexen Hydriden, bevorzugt Natriumborhydrid in Gegenwart eines polaren Lösungsmittels, z. B. eines Alkohols bevorzugt Methanol oder Ethanol bei Temperaturen zwischen 0 und 80 °C oder $C_2$) durch Einwirkung von Wasserstoff in Gegenwart eines Hydrierkatalysators wie Platin oder Raney-Nickel und in Gegenwart eines polaren Lösungsmittels wie Methanol, Ethanol oder Ethylacetat bei Temperaturen zwischen 20 und 100 °C und Drucken von 1 bis 100 bar zu Verbindungen der Formel XII

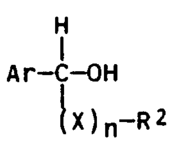

XII

die dann in einer zweiten Stufe in Verbindungen der Formel II überführt werden und zwar, wenn

a) Z für Chlor steht, durch Umsetzung mit Thionylchlorid ggf. in Gegenwart eines Lösungmittels wie Methylenchlorid oder Chloroform bei Temperaturen zwischen 20 bis 70 °C oder wenn

b) Z für Brom steht, durch Umsetzung mit Phosphortribromid ggf. in Gegenwart eines Lösungsmittels wie Methylenchlorid oder Chloroform bei Temperaturen zwischen 0 und 70 °C, vorzugsweise 20-30 °C oder wenn

c) Z für Methan-, Benzol- oder 4-Tolyl-sulfonat steht, durch Umsetzung mit den entsprechenden Sulfonsäurechloriden oder anhydriden ggf. in Gegenwart einer organischen Base wie Triethylamin, Dimethylcyclohexylamin, Diethylcyclohexylamin, Pyridin, 4-N,N-Dimethylaminopyridin und ggf. in Gegenwart eines Verdünnungsmittels wie Methyl-tert.-Butylether, Diethylether, Tetrahydrofuran, Methylenchlorid, Chloroform, Pentan, Hexan, Cyclohexan oder Toluol bei Temperatur zwischen 20 und 150 °C.

Verbindungen der Formel II, in denen X für $CH_2$, $R^1$ für Nitril, n für 0 oder 1 und Z für Chlor steht erhält man z. B.

a) Durch Umsetzung Von Ketonen mit Titantetrachlorid in Gegenwart von Trimethylsilylcyanid (TMS-CN) (s. z. B. S. Kiyooka, R. Fujiyama, K. Kawaguchi Chem. Lett. 1979-80 [1984]) nach dem Schema

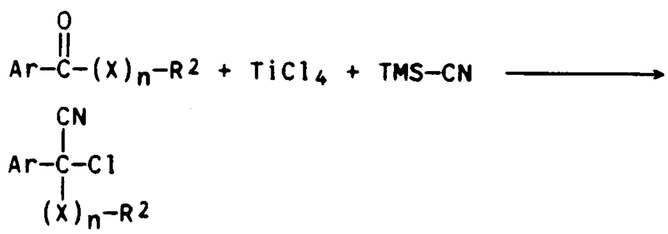

oder

b) durch Chlorierung von Nitrilen mit Tetrachlorkohlenstoff in Gegenwart von 50 %iger Natronlauge unter Phasentransferbedingungen (PTC) (s. z. B. A. Jonczyk, A. Kwast, M. Makosza, Z. Org. Chem. 44, 1192-4, [1979]) nach dem Schema

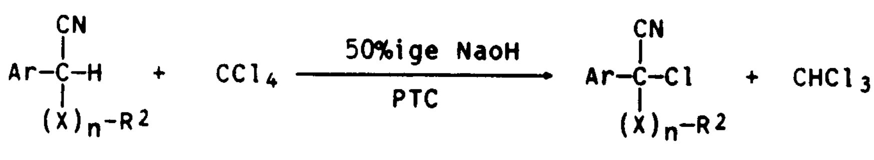


Die folgenden Beispiele beschreiben die Herstellung der neuen Verbindungen:

Beispiel 1

a) Herstellung von 1-Hydroxy-1,2,4-triazol und seines Kaliumsalzes

103,5 g (1,5 mol) 1-H-1,2,4-Triazol wurden in 1344 g (12 mol) 50 %igem wäßrigem Kaliumhydroxid gelöst. Unter Eiskühlung wurden 340 g (3 mol) 30 %iges $H_2O_2$, portionsweise 555 g (3,75 mol) Phthalsäureanhydrid zugegeben und 2 Stunden bei Raumtemperatur (20 bis 30 °C) gerührt. Anschließend wurde mit ca. 35 %iger Schwefelsäure auf einen pH-Wert unter 1,5 angesäuert, der entstandene Niederschlag abgesaugt und das Filtrat durch quantitative Hochdruckflüssigkeitschromatographie (HPLC)-Messung untersucht. Man erhielt 65 g (51 %), das wie üblich aufgearbeitet wurde; Fp.: 132 °C.

10 g 1-Hydroxy-1,2,4-triazol (117 mmol) werden mit 300 ml Dimethylformamid gelöst. Anschließend gibt man 13,2 g (117 mmol) Kaliummethanolat (50 %) und 100 ml Methanol hinzu. Das Gemisch wird erhitzt und das MeOH/$H_2O$-Gemisch azeotrop herausdestilliert. Man gibt nochmals 50 ml MeOH hinzu und destilliert das Gemisch mit Wasser nochmals azeotrop ab.

Die Lösung des Kaliumsalzes von 1-Hydroxy-1,2,4-triazol in DMF kann entweder direkt weiter umgesetzt werden, oder man dampft das Lösemittel vollständig ab und erhält einen farblosen Niederschlag, der weiterverwendet wird.

b) Herstellung von Di-(4-fluorphenyl)-chlormethan

Zu 33 g 4,4'-Difluordiphenylmethylcarbinol gelöst in 150 ml Dichlormethan gibt man 15 g wasserfreies, pulverisiertes Calciumchlorid und leitet in die Mischung unter Rühren bei 5-10 °C trockenen Chlorwasserstoff ein. Nach 2 h wird vom $CaCl_2$ abdekantiert und die Lösung i. Vak. eingeengt. Man erhält 34 g Di-(4-fluorphenyl)-chlormethan als blaßgelbliche Flüssigkeit.

c) Herstellung von Di-(4-fluorphenyl)-(1,2,4-triazol-1-yl-1-oxy-)-methan (Verbindung Nr. 1)

Zu 1,5 g Kaliumsalz des 1-Hydroxy-1,2,4-triazols in 100 ml Dimethylformamid gibt man 100 mg Kaliumiodid und 2,9 g Di-(4-fluorphenyl)-chlormethan und rührt 2 h bei Raumtemperatur. Nach Abdampfen des Lösungsmittels i. Vak. wird der Rückstand mit 50 ml Methyl -tert. -butylether auf genommen und zweimal mit Wasser ausgeschüttelt. Nach Einengen der organischen Phase bleiben 3,3 g eines blaßgelben Öls, das über eine kurze Kieselgelsäule mit Dichlormethan chromatographiert wird. Ausbeute 2,0 g helles Öl.
$^1$H-NMR (300 MHz, $CDCl_3$): 6.5 ppm (1H, s); 7.0-7.4 ppm (8H, m); 7.5 ppm (1H, s); 7.8 ppm (1H, s)

Beispiel 2

Herstellung von 2,4-Dichlorbenzyl-phenyl-thio-(1,2,4-triazol-1-yl-1-oxy-)-methan (Verbindung Nr. 2)

Man rührt 26.9 g 2,4-Dichlorbenzyl-phenyl-sulfid und 13.4 g N-Chlorsuccinimid in 100 ml Tetrachlormethan bei 0 °C, 2 h lang, läßt den Ansatz sich dann auf Raumtemperatur erwärmen und rührt weitere 48 h. Danach wird das in der Mischung suspendierte Succinimid abfiltriert und das Filtrat i. Vak. vom Lösungsmittel befreit. Zurück bleiben 30,3 g $\alpha$-chlor-2,4-dichlorbenzyl-phenyl-sulfid als Öl.

4,6 g dieses Öls werden zu einer Mischung von 1,5 g Kaliumsalz des 1-Hydroxy-1,2,4-triazols und 0,3 g Kaliumiodid in 30 ml DMF getropft. Laut Dünnschichtchromatographie (DC)-Kontrolle ist die Reaktion nach 2stündigem Rühren bei Raumtemperatur beendet. Man dampft das DMF i. Vak. ab, versetzt den Rückstand mit 50 ml Wasser und extrahiert dreimal mit je 50 ml Methyl-tert.-butylether. Die vereinigten organischen Lösungen werden mit 30 ml Wasser gewaschen, über Magnesiumsulfat getrocknet, i. Vak. eingeengt (4.7 g Öl) und anschließend an einer Kieselgelsäule mit Diethylether als Laufmittel chromatographiert. Nach 0,1 g Vorlauffraktion, die verworfen werden, erhält man 1,0 g 2,4-Dichlorbenzylphenylthio-(1,2,4-triazol-1-yl-1-oxy)-methan als blaßgelbes Öl.
$^1$H-HMR (300 MHz, $CDCl_3$): 6.7 ppm (1H, s); 7.1-7.6 ppm (8H, m); 7.9 ppm (1H, s); 8.5 ppm (1H, s).

Beispiel 3

a) 2-Chlor-2,2-di-(4-fluorphenyl)-acetonitril

Unter Stickstoffgas werden 4.4 g (20 mmol) 4,4' Difluorbenzophenon in 50 ml $CH_2Cl_2$ gelöst. Nacheinander werden dann bei 0 °C 2.7 ml (24 mmol) Titantetrachlorid und 2,7 ml (20 mmol) Trimethylsilylcyanid zugegeben. Nach 30 min läßt man sich die Mischung auf Raumtemperatur erwärmen und rührt noch 24 h nach. Nach Zugabe von wäßriger $NaHCO_3$-Lösung wird mit Ether extrahiert. Die Etherphase wird über $Na_2SO_4$ getrocknet. Das Produkt wird als gelbes Öl gewonnen.
Ausbeute: 5 g
IR: 1602 $cm^{-1}$, 1508 $cm^{-1}$, 1239 $cm^{-1}$, 1163 $cm^{-1}$, 835 $cm^{-1}$

b) Cyano-di-(4-fluorphenyl)-(1,2,4-triazol-1-yl-1-oxy-)-methan (Verbindung Nr. 3)

Unter Stickstoff werden 1,5 g (12 mmol) N-Hydroxytriazol-Kaliumsalz in 20 ml abs. DMF vorgelegt. In diese Lösung tropft man bei leicht exothermer Reaktion eine DMF-Lösung von 3.16 (12 mmol) 2-Chloro-2,2-di(4-Fluorphenyl) acetonitril. Es wird noch 3 h bei Raumtemperatur nachgerührt. Die Reaktionslösung wird eingeengt auf Wasser gegeben und mit Essigsäureethylester extrahiert. Das Produkt wird aus Essigester als blaßgelbes Öl gewonnen, das durch Verrühren mit Diisopropylether auskristallisiert.
Ausbeute: 2.5 g
Schmelzpunkt 124 °C H-NMR: 7.15 ppm, t (4H); 7.4-7.6 ppm, m (4 H); 7.72 ppm, s (1H); 7.78 ppm, s (1H)

Beispiel 4

1-(2,4-Dichlorphenyl)-1-(1,2,4-triazol-1-yl-1-oxy)-butan (Verbindung Nr. 33)

1.2 g (10 mmol) 1-Hydroxi-1,2,4-triazol-Kaliumsalz wurden in 100 ml DMF gelöst und bei Raumtemperatur zu einer Lösung von 4.0 g (10 mmol) 1-Brom-1-(2,4-dichlorphenyl)-butan in 50 ml DMF gegeben. Die Mischung wurde mit 0,1 g Kaliumjodid versetzt und 10 Minuten auf Rückflußtemperatur erwärmt. Danach wurde das Lösemittel im Vakuum entfernt, der Rückstand in Ether aufgenommen und 3 mal mit Wasser gewaschen. Nach Trocknen der org. Phase mit $Na_2SO_4$ wurde das Lösemittel im Vakuum entfernt.
Ausbeute: 2.7 g braunes Öl (94 %), das säulenchromatogr. gereinigt wurde (Kieselgel, Essigester: Cyclohexan (1:1))

Tabelle

In entsprechender Weise lassen sich die folgenden Verbindungen der allgemeinen Formel I herstellen:

| Beispiel Nr. | Ar | $R^1$ | $(X)_n$ | $R^2$ | $^1H$-NMR (ppm) Fp ($^oC$) bzw. JR ($cm^{-1}$) |
|---|---|---|---|---|---|
| 1 | 4-Fluorphenyl | H | - | 4-Fluorphenyl | s. Bsp. 1 |
| 2 | 2,4-Dichlorphenyl | H | S | Phenyl | s. Bsp. 2 |
| 3 | 4-Fluorphenyl | CN | - | 4-Fluorphenyl | s. Bsp. 3 |
| 4 | 4-Fluorphenyl | H | - | 2-Chlorphenyl | 1510, 1235, 1003, 832, 757, 681 $cm^{-1}$ |
| 5 | 4-Fluorphenyl | H | - | 2-Fluorphenyl | 1511, 1491, 1234, 1003, 760, 681 $cm^{-1}$ |
| 6 | 4-Fluorphenyl | H | S | 2-Chlorphenyl | 1509, 1451, 1233, 1226, 1125, 1005, 891, 843, 766, 679 $cm^{-1}$ |
| 7 | 4-Fluorphenyl | H | $CH_2$ | 2-Chlorphenyl | 1606, 1511, 1436, 1228 1004, 838, 754, 681 $cm^{-1}$ |

| Beispiel Nr. | Ar | $R^1$ | $(X)_n$ | $R^2$ | $^1H$-NMR (ppm) Fp (°C) bzw. JR ($cm^{-1}$) |
|---|---|---|---|---|---|
| 8 | Phenyl | H | - | 4-Fluorphenyl | 1511, 1275, 1229, 1004, 832, 699, 681 $cm^{-1}$ |
| 9 | 2-Fluorphenyl | H | - | Phenyl | 1490, 1452, 1274, 1241, 1003, 943, 762, 698, 681 $cm^{-1}$ |
| 10 | 3-Trifluormethylphenyl | H | - | Phenyl | 1333, 1168, 1127, 1075, 701, 682 $cm^{-1}$ |
| 11 | 4-Chlorphenyl | H | - | 2,2-Dichlorvinyl | 73-75 °C |
| 12 | 2,4-Dichlorphenyl | H | S | 4-Chlorphenyl | 85-87 °C |
| 13 | 2,4-Dichlorphenyl | H | S | Ethyl | 1589, 1497, 1472, 1275, 866, 856 $cm^{-1}$ |
| 14 | 4-Fluorphenyl | H | - | 2-Trifluormethylbenzyl | |
| 15 | 4-Fluorphenyl | H | - | 2-Brombenzyl | |
| 16 | 4-Fluorphenyl | H | - | 2-Methylbenzyl | |
| 17 | Phenyl | H | - | 2-Chlorphenyl | 1490, 1270, 1130, 1050, 748 $cm^{-1}$ |

| Beispiel Nr. | Ar | $R^1$ | $(X)_n$ | $R^2$ | $^1H$-NMR (ppm) Fp ($^oC$) bzw. JR ($cm^{-1}$) |
|---|---|---|---|---|---|
| 18 | Phenyl | H | $CH_2$ | 2-Chlorphenyl | |
| 19 | Phenyl | H | - | 2-(4-Chlorphenyl)ethyl | |
| 20 | 4-Phenylphenyl | H | - | tert.-Butyl | |
| 21 | 4-Phenylphenyl | H | - | n-Butyl | |
| 22 | 4-Phenoxyphenyl | H | - | n-Hexyl | |
| 23 | 4-Phenoxyphenyl | H | S | tert.-Butyl | |
| 24 | 4-Phenylphenyl | H | S | tert.-Butyl | |
| 25 | 4-Chlorphenyl | H | S | tert.-Butyl | |
| 26 | 4-Chlorphenyl | H | $CH_2$ | tert.-Butyl | |
| 27 | 2-Chlor-4-(4-chlorphenoxy)-phenyl | H | S | tert.-Butyl | |
| 28 | 2-Chlor-4-(4-chlorphenoxy)-phenyl | H | S | Ethyl | |
| 29 | 2-Chlor-4-(4-chlorphenoxy)-phenyl | H | S | Methyl | |
| 30 | 2-Chlor-4-(4-chlorphenoxy)-phenyl | H | S | 2-Methylpropyl | |
| 31 | 4-Chlorphenyl | H | - | 2-Cyclopropyl-ethyl-(2) | |
| 32 | 4-Methylphenyl | H | - | 2-Cyclopropyl-ethyl-(2) | |
| 33 | 2,4-Dichlorphenyl | H | - | n-Propyl | 1510, 1470, 1380, 1273, 1240, 1100, 965, 680 $cm^{-1}$ |

| Beispiel Nr. | Ar | $R^1$ | $(X)_n$ | $R^2$ | $^1H$-NMR (ppm) Fp (°C) bzw. JR ($cm^{-1}$) |
|---|---|---|---|---|---|
| 34 | 2,4-Dichlorphenyl | H | - | n-Hexyl | |
| 35 | 2,4-Dichlorphenyl | H | S | Methyl | |
| 36 | 1-Naphthyl | H | S | n-Propyl | |
| 37 | 2-Naphthyl | H | S | n-Butyl | |
| 38 | 2,4-Dichlorphenyl | H | S | Cyclohexyl | |
| 39 | 2-Chlor-4-(4-chlorphenoxy)-phenyl | H | - | Ethyl | |
| 40 | 2-Chlor-4-(4-chlorphenoxy)-phenyl | H | - | n-Butyl | |
| 41 | 4-(4-chlorphenoxy)-phenyl | H | - | n-Propyl | |
| 42 | 2-Thienyl | H | - | 2-Chlorphenyl | |
| 43 | 2-Thienyl | H | S | 4-Chlorphenyl | |
| 44 | 2-Thienyl | H | $CH_2$ | 2-Bromphenyl | |
| 45 | 3-Thienyl | H | - | 2-Trifluormethylphenyl | |
| 46 | 3-Thienyl | H | S | 2-Chlorphenyl | |
| 47 | 2-Pyridyl | H | - | 2-Chlorphenyl | |
| 48 | 2-Pyridyl | H | - | 4-Chlorphenyl | |
| 49 | 4-Chlor-2-pyridyl | H | - | 2-Chlorphenyl | |
| 50 | 4-Chlor-2-pyridyl | H | - | 4-Fluorphenyl | |
| 51 | 4-Chlor-2-pyridyl | H | - | n-Propyl | |
| 52 | 4-Chlor-2-pyridyl | H | - | 2-Methylpropyl | |
| 53 | 4-Chlor-2-pyridyl | H | S | n-Butyl | |
| 54 | 4-Methoximino-phenyl | H | - | n-Butyl | |

| Beispiel Nr. | Ar | $R^1$ | $(X)_n$ | $R^2$ | $^1H$-NMR (ppm) Fp ($^oC$) bzw. JR ($cm^{-1}$) |
|---|---|---|---|---|---|
| 55 | 4-Ethoximinophenyl | H | S | tert.-Butyl | |
| 56 | 4-Isopropoximino | H | S | tert.-Butyl | |
| 57 | 4-Methoximino | H | S | Ethyl | |
| 58 | 4-Methoximino | H | S | n-Hexyl | |
| 59 | 4-Methoximino | H | S | 2-Chlorphenyl | |
| 60 | 4-Ethoximino | H | - | Phenyl | |
| 61 | 4-n-Butoximino | H | S | n-Butyl | |
| 62 | 4-Methoximino | H | $CH_2$ | Phenyl | |
| 63 | 4-Methoximino | H | $CH_2$ | 2-Chlorphenyl | |
| 64 | 4-Methoximino | H | $CH_2$ | tert.-Butyl | |
| 65 | Phenyl | H | $CH_2$ | 2,4-Dichlorphenyl | |
| 66 | 4-Fluorphenyl | H | $CH_2$ | 4-Chlorbenzyl | |
| 67 | 2,4-Dichlorphenyl | H | $CH_2$ | n-Propyl | |
| 68 | Phenyl | H | $CH_2$ | 3-Phenylpropyl-(1) | |
| 69 | Phenyl | H | O | Phenyl | |
| 70 | Phenyl | H | O | 2-Chlorphenyl | |
| 71 | Phenyl | H | O | Benzyl | |
| 72 | Phenyl | H | O | tert.-Butyl | |
| 73 | 4-Fluorphenyl | H | O | tert.-Butyl | |
| 74 | 4-Chlorphenyl | H | O | tert.-Butyl | |
| 75 | 2,4-Dichlorphenyl | H | O | n-Butyl | |
| 76 | 2,4-Dichlorphenyl | H | O | 2-Methylpropyl | |
| 77 | 2,4-Dichlorphenyl | H | O | tert.-Butyl | |

| Beispiel Nr. | Ar | $R^1$ | $(X)_n$ | $R^2$ | $^1H$-NMR (ppm) Fp ($^oC$) bzw. JR ($cm^{-1}$) |
|---|---|---|---|---|---|
| 78 | 4-Fluorphenyl | H | O | 2-chlorphenyl | |
| 79 | 4-Fluorphenyl | H | O | 2,6-Dichlorphenyl | |
| 80 | 4-Fluorphenyl | H | O | 2,4-Dichlorphenyl | |
| 81 | 4-Phenylphenyl | H | O | n-Butyl | |
| 82 | 4-tert.-Butyl-phenyl | H | S | tert.-Butyl | |
| 83 | 4-tert.-Butylphenyl | H | S | n-Butyl | |
| 84 | Phenyl | H | - | 4-tert.-Butylphenyl | |
| 85 | 2,4-Dichlorphenyl | H | - | 4-Methoxyphenyl | |
| 86 | 4-Fluorphenyl | H | - | 2-Methoxyphenyl | |
| 87 | 4-Fluorphenyl | H | - | 2-Phenoxyethyl | |
| 88 | 4-Fluorphenyl | H | - | 4-Phenoxybutyl | |
| 89 | Phenyl | H | - | 3-Phenoxypropyl | |
| 90 | Phenyl | H | - | 2-(2-Chlorphenoxy)ethyl-(1)- | |
| 91 | 4-Fluorphenyl | H | - | 3-(2-Fluorphenoxy-)propyl-(1)- | |
| 92 | 4-Fluorphenyl | H | - | 2-Pyridyl | |
| 93 | Phenyl | H | - | 4-Pyridyl | |
| 94 | Phenyl | H | - | 2-Thienyl | |
| 95 | 4-Fluorphenyl | H | - | 3-Thienyl | |
| 96 | 4-Fluorphenyl | H | - | 4-Chlor-2-pyridyl | |
| 97 | 4-Fluorphenyl | H | - | 3-Chlor-4-pyridyl | |
| 98 | 4-Fluorphenyl | H | S | 2-Pyridyl | |
| 99 | 4-Trifluormethylphenyl | H | S | 2-Bromphenyl | |
| 100 | 2,4-Dichlorphenyl | H | S | 3-Propinyl-1 | |

| Beispiel Nr. | Ar | $R^1$ | $(X)_n$ | $R^2$ | $^1H$-NMR (ppm) Fp ($^oC$) bzw. JR ($cm^{-1}$) |
|---|---|---|---|---|---|
| 101 | 2,4-Dichlorphenyl | H | - | Ethinyl | |
| 102 | 2,4-Dichlorphenyl | H | - | Vinyl | |
| 103 | 4-Phenylphenyl | H | - | Propenyl | |
| 104 | 4-Phenylphenyl | H | S | 2,3,3-Trichlorpropenyl | |
| 105 | Phenyl | CN | - | 4-Fluorphenyl | |
| 106 | Phenyl | CN | - | 2-Chlorphenyl | |
| 107 | Phenyl | CN | - | 2,4-Dichlorphenyl | |
| 108 | 4-Fluorphenyl | CN | - | 2-Chlorphenyl | 1509, 1238, 985, 760, 680 |
| 109 | 4-Fluorphenyl | CN | - | 4-Chlorphenyl | |
| 110 | 4-Fluorphenyl | CN | - | 2-Chlorbenzyl | |
| 111 | 4-Fluorphenyl | CN | - | n-Butyl | |
| 112 | 4-Fluorphenyl | CN | - | tert.-Butyl | |
| 113 | 2,4-Dichlorphenyl | CN | - | Ethyl | |
| 114 | 2,4-Dichlorphenyl | CN | - | n-Butyl | |
| 115 | 2,4-Dichlorphenyl | CN | - | 2-Methylpropyl | |
| 116 | 4-Chlorphenyl | CN | - | 2-Cyclopropylethyl-(2) | |
| 117 | 4-Fluorphenyl | CN | - | 2-Cyclopropylehtyl-(2) | |
| 118 | 4-Chlorphenyl | CN | - | tert.-Butyl | |
| 119 | 2,4-Dichlorphenyl | CN | - | n-Hexyl | |
| 120 | 4-Fluorphenyl | CN | - | 2-Trifluormethylbenzyl | |
| 121 | 2-Chlor-4-(4-chlor-phenoxy)-phenyl | CN | - | Ethyl | |
| 122 | 2-Chlor-4-(4-chlor-phenoxy)-phenyl | CN | - | n-Butyl | |

| Beispiel Nr. | Ar | $R^1$ | $(X)_n$ | $R^2$ | $^1H$-NMR (ppm) Fp (°C) bzw. JR ($cm^{-1}$) |
|---|---|---|---|---|---|
| 123 | 4-Fluorphenyl | CN | - | 2-Fluorphenyl | 1510, 1491, 1454, 1239, 996, 837, 763, 680, $cm^{-1}$ |
| 124 | 4-Chlorphenyl | CN | - | 2-Chlorphenyl | 1492, 1274, 1095, 995, 824, 760, 680 $cm^{-1}$ |
| 125 | Phenyl | H | - | Phenyl | |
| 126 | 4-Chlorphenyl | H | - | 2-Chlorphenyl | |
| 127 | 4-Chlorphenyl | H | - | Phenyl | 1493, 1275, 1239, 1088, 940, 680 $cm^{-1}$ |
| 128 | 4-Chlorphenyl | H | - | 4-Chlorphenyl | 1493, 1275, 1241, 1092, 820, 805, 679 $cm^{-1}$ |
| 129 | 4-Chlorphenyl | H | - | n-Propyl | 1490, 1275, 1240, 1090, 945, 870, 680 $cm^{-1}$ |
| 130 | 4-Fluorphenyl | H | - | n-Propyl | 1510, 1275, 1225, 1005, 835, 680 $cm^{-1}$ |
| 131 | 4-Chlorphenyl | H | - | 4-Methylbutyl | 1492, 1270, 1090, 940, 825, |
| 132 | 4-Chlorphenyl | H | - | 3-Isopropylisoxazol-5-yl | |
| 133 | 4-Fluorphenyl | H | - | 3-Isopropylisoxazol-5-yl | 3.07 (sept,1H), 6.2 (s,1H), 6.5 (s,1H), 7.68 (s,1H), 7.75 (s,1H) ppm) |
| 134 | 2,4-Dichlorphenyl | H | - | 3-Pyridyl | |
| 135 | 4-Fluorphenyl | H | - | 3-Pyridyl | |
| 136 | 4-Fluorphenyl | H | - | 1,2,4-Triazol-1-yl | |
| 137 | 4-Methoximinophenyl | H | - | 4-Fluorphenyl | |
| 138 | Phenyl | CN | - | 2-(4-Chlorphenyl)ethyl | 7.1 (d,2H), 7.26 (d,2H), 7.62 (s,1H), 7.68 (s,1H) ppm |

| Beispiel Nr. | Ar | $R^1$ | $(X)_n$ | $R^2$ | |
|---|---|---|---|---|---|
| 139 | 4-Fluorphenyl | CN | - | 2-(4-Chlorphenyl)ethyl | 2.45-3.08(m,4H); 7.65(s,1H); 7.8(s,1H) |
| 140 | 4-Chlorphenyl | CN | - | " | |
| 141 | 2,4-Dichlorphenyl | CN | - | " | 2.65-3.02(m,4H); 7.69(s,1H); 8.13(s,1H) |
| 142 | Phenyl | CN | - | 2-(4-Fluorphenyl)ethyl | 2.5-3.04(m,4H); 7.66(s,1H); 7.74(s,1H) |
| 143 | 4-Fluorphenyl | CN | - | " | 2.5-3.06(m,4H); 7.65(s,1H); 7.85(s,1H) |
| 144 | 4-Chlorphenyl | CN | - | " | 2.48-3.04(m,4H); 7.66(s,1H); 7.86(s,1H) |
| 145 | 2,4-Dichlorphenyl | CN | - | " | 2.65-3.05(m,4H); 7.68(s,1H); 8.14(s,1H) |
| 146 | Phenyl | CN | - | 2-(Phenyl)ethyl | 2.5-3.1(m,4H); 7.65(s,1H); 7.82(s,1H) |
| 147 | 4-Fluorphenyl | CN | - | " | 2.66-3.08(m,4H); 7.7(s,1H); 8.15(s,1H) |
| 148 | 4-Chlorphenyl | CN | - | " | |
| 149 | 2,4-Dichlorphenyl | CN | - | " | 2.66-3.08(m,4H); 7.7(s,1H); 8.15(s,1H) |
| 150 | Phenyl | CN | - | 2(2,4-Dichlorphenyl)ethyl | |
| 151 | 4-Fluorphenyl | CN | - | " | |
| 152 | 4-Chlorphenyl | CN | - | " | |
| 153 | 2,4-Dichlorphenyl | CN | - | " | |
| 154 | 4-Fluorphenyl | H | - | 2,4-Dichlorphenyl | 6.77(s,1H); 7.62(s,1H); 7.77(s,1H) |
| 155 | 4-Fluorphenyl | H | - | 3-(p-tert.-Butylphenyl)-isoxazol-5-yl | 1.35(s,9H); 6.6(s,1H); 6.65(s,1H); 7.72(s,1H); 7.78(s,1H) |
| 156 | 4-Fluorphenyl | H | - | 3-tert.-Butylisoxazol-5-yl | 1.32(s,9H); 6.25(s,1H); 6.54(s,1H); 7.66(s,1H); 7.76(s,1H) |
| 157 | 4-Fluorphenyl | H | - | 3-n-Propylisoxazol-5-yl | 6.2(s,1H); 6.55(s,1H); 7.72(s,1H); 7.78(s,1H) |
| 158 | 4-Fluorphenyl | H | - | 3-Cyclopentylisoxazol-5-yl | 6.16(s,1H); 6.52(s,1H); 7.68(s,1H); 7.77(s,1H) |
| 159 | 4-Fluorphenyl | H | - | 3-sec.-Butylisoxazol-5-yl | 6.17(s,1H); 6.53(s,1H); 7.66(s,1H); 7.77(s,1H) |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze. Es werden die Pilze oder die durch Pilzbefall bedrohten Pflanzen, Saatgüter, Materialien oder der Erdboden mit einer fungizid wirksamen Menge des Wirkstoffs behandelt.

Die neuen Verbindungen können praktisch alle Entwicklungsstadien einer Pflanze verschiedenartig beeinflussen und werden deshalb als Wachstumsregulatoren eingesetzt. Die Wirkungsvielfalt der Pflanzenwachstumsregulatoren hängt ab vor allem

a) von der Pflanzenart und -Sorte,
b) von dem Zeitpunkt der Applikation, bezogen auf das Entwicklungsstadium der Pflanze und von der Jahreszeit,
c) von dem Applikationsort und -verfahren (Samenbeize, Bodenbehandlung oder Blattapplikation),
d) von klimatischen Faktoren, z.B. Temperatur, Niederschlagsmenge, außerdem auch Tageslänge und Lichtintensität,
e) von der Bodenbeschaffenheit (einschließlich Düngung),
f) von der Formulierung bzw. Anwendungsform des Wirkstoffs und
g) von den angewendeten Konzentrationen der aktiven Substanz.

Aus der Reihe der verschiedenartigen Anwendungsmöglichkeiten der erfindungsgemäßen Pflanzenwachstumsregulatoren im Pflanzenanbau, in der Landwirtschaft und im Gartenbau, werden einige nachstehend erwähnt.

A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.

Als vorteilhaft für die Praxis erweist sich z.B. die Verringerung des Grasbewuchses an Straßenrändern, Hecken, Kanalböschungen und auf Rasenflächen wie Park-, Sport- und Obstanlagen, Zierrasen und Flugplätzen, so daß der arbeits- und kostenaufwendige Rasenschnitt reduziert werden kann.

Von wirtschaftlichem Interesse ist auch die Erhöhung der Standfestigkeit von lageranfälligen Kulturen wie Getreide, Reis, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.

Von praktischer Bedeutung ist auch die Reduzierung des vegetativen Wachstums bei Obstbäumen und anderen Gehölzen, da so Schnittkosten eingespart werden können.

Wichtig ist auch die Anwendung zur Hemmung des Längenwachstums und zur zeitlichen Veränderung des Reifeverlaufs bei Baumwolle. Damit wird ein vollständig mechanisiertes Beernten dieser

wichtigen Kulturpflanze ermöglicht.

Durch Anwendung der neuen Verbindungen kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit den neuen Verbindungen läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum übergang in die generative Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag, bezogen auf die Bodenfläche, erzielt werden kann.

B. Mit den neuen Mitteln lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Die Wirkstoffe der Formel I können Ertragssteigerungen durch Eingriffe in den pflanzlichen Stoffwechsel bzw. durch Förderung oder Hemmung des vegetativen und/oder des generativen Wachstums verursachen.

C. Mit den Verbindungen der Formel I lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Die Förderung der Ausbildung eines Trenngewebes zwischen der Blatt- und Sproßachse ist auch für ein gut kontrollierbares Entblättern von Nutzpflanzen, z.B. Baumwolle, wesentlich.

D. Mit den Wirkstoffen kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.

- die Öffnungsweite der Stomata reduziert wird
- eine dickere Epidermis und Cuticula ausgebildet werden
- die Durchwurzelung des Bodens verbessert wird
- das Mikroklima im Pflanzenbestand durch einen kompakteren Wuchs günstig beeinflußt wird.

Die erfindungsgemäß zu verwendenden Wirkstoffe können den Kulturpflanzen sowohl vom Samen her (als Saatgutbeizmittel) als auch über den Boden, d.h. durch die Wurzel sowie durch Spritzung über das Blatt zugeführt werden.

Infolge der hohen Verträglichkeit der Pflanzen für die Wirkstoffe kann die Aufwandmenge als Wachstumsregulatoren stark variiert werden. Bei der Saatgutbehandlung werden im allgemeinen Wirkstoffmengen von 0,001 bis 50 g je Kilogramm Saatgut, vorzugsweise 0,005 bis 0,5 g, benötigt. Für die Blatt- und Bodenbehandlung sind im allgemeinen Gaben von 0,01 bis 10 kg/ha, bevorzugt 0,05 bis 1 kg/ha ausreichend.

Die Mittel können in Form üblicher Formulierungen angewendet werden wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen

dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin-Sulfitablaugen und Methylcellulose.

Die fungiziden Mittel und wachstumsregulierenden Mittel enthalten im allgemeinen zwischen 0,1 und 95, vorzugsweise zwischen 0,5 und 90 Gew.% Wirkstoff.

Die Aufwandmengen als Fungizide liegen je nach Art des gewünschten fungiziden Effektes zwischen 0,02 und 3 kg Wirkstoff oder mehr je ha. Die neuen Verbindungen können auch im Materialschutz (Holzschutz) eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 1 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 3 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 4 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 5 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 6 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 7 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 9 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 17 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 33 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N'-propylen-bis-dithiocarbamat),
Zink-(N,N'-propylen-bis-dithiocarbamat),
N,N'-Polypropylen-bis-(thiocarbamoyl)-disulfid;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;
5-Nitro-isophthalsäure-di-isopropylester
heterocyclische Substanzen, wie
2-Heptadecyl-2-imidazolin-acetat,
2,4-Dichlor-6-(o-chloranilino)-s-triazin,
O,O-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon,
2-Thio-1,3-dithiolo[4,5-b]chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,
N-Dichlorfluormethylthio-N',N'-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydroazano)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilid,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N'-imidazol-yl-harnstoff,

1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorophenyl)-α-(4-chlorophenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
sowie verschiedene Fungizide, wie
Dodecylguanidinacetat,
3-[3-(3,5-Dimethyl-2-oxycyclohexyl)-2-hydroxyethyl]-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion, 3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol,
N-(3-Chlor-2,6-dinitro-4-trifluormethyl-phenyl)-5-trifluormethyl-3-chlor-2-aminopyridin,
1-((bis-(4-Fluorphenyl)-methylsilyl)-methyl)-1H-1,2,4-triazol.

Anwendungsbeispiele

Als Vergleichswirkstoff wurde 1-Bis-(4-fluorophenyl)-methyl-1H-1,2,4-triazol (A) - bekannt aus EP 165 777 - benutzt.

Anwendungsbeispiel 1

Wirksamkeit gegen weizenmehltau

Blätter von in Töpfen gewachsenen Weizenkeimlingen der Sorte "Kanzler" wurden mit wäßriger Spritzbrühe, die 80 Gew.-% Wirkstoff und 20 % Emulgiermittel in der Trockensubstanz enthielten, besprüht und 24 Stunden nach dem Antrocknen des Spritzbelages mit Oidien (Sporen) des Weizenmehltaus (Erysiphe graminis var. tritici) bestäubt. Die Versuchspflanzen wurden anschließend im Gewächshaus bei Temperaturen zwischen 20 und 22°C und 75 bis 80 % relativer Luftfeuchtigkeit aufgestellt. Nach 7 Tagen wurde das Ausmaß der Mehltauentwicklung ermittelt.

Das Ergebnis zeigt, daß die Wirkstoffe 1, 3, 4, 5, 6, 7, 9, 17, 33, 108 123, 124, 127, 128 und 129 bei der Anwendung als 0,006 %ige (Gew.%) Spritzbrühe eine bessere fungizide Wirkung zeigen (95 %) als der bekannte Vergleichswirkstoff A (75 %).

Anwendungsbeispiel 2

Wirksamkeit gegen Weizenbraunrost

Blätter von in Töpfen gewachsenen Weizensämlingen der Sorte "Kanzler" wurden mit Sporen des Braunrostes (Puccinia recondita) bestäubt. Danach wurden die Töpfe für 24 Stunden bei 20 bis 22°C in eine Kammer mit hoher Luftfeuchtigkeit (90 bis 95 %) gestellt. Während dieser Zeit keimten die Sporen aus und die Keimschläuche drangen in das Blattgewebe ein. Die infizierten Pflanzen wurden anschließend mit wäßrigen Spritzbrühen, die 80 % Wirkstoff und 20 % Emulgiermittel in der Trockensubstanz enthielten, tropfnaß gespritzt. Nach dem Antrocknen des Spritzbelages wurden die Versuchspflanzen im Gewächshaus bei Temperaturen zwischen 20 und 22° und 65 bis 70 % relativer Luftfeuchte aufgestellt. Nach 8 Tagen wurde das Ausmaß der Rostpilzentwicklung auf den Blättern ermittelt.

Das Ergebnis zeigt, daß die Wirkstoffe 1, 4, 7, 127 und 128 bei der Anwendung als 0,025 %ige Spritzbrühe eine bessere fungizide Wickung zeigen (95 %) als der bekannte Vergleichswirkstoff A (30 %).

## Patentansprüche

**1.** Verbindungen der Formel I

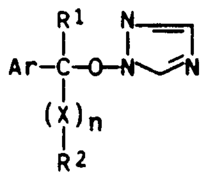

I

in welcher

Ar für gegebenenfalls ein- oder mehrfach durch Halogen, $C_1$-$C_4$-Alkoxy, $C_1$-$C_4$-Alkyl, Trifluormethyl, $C_1$-$C_4$-Alkoximino, gegebenenfalls durch Halogen oder Trifluormethyl substituiertes Phenyl oder Phenoxy substituiertes Phenyl, gegebenenfalls durch die gleichen Reste substituiertes Pyridyl, gegebenenfalls durch die gleichen Reste substituiertes Thienyl oder gegebenenfalls durch die gleichen Reste substituiertes Naphthyl steht,

$R^1$ für Wasserstoff steht oder für den Fall, daß n = 0 ist, zusätzlich für CN steht,

$R^2$ für gegebenenfalls ein- oder mehrfach durch Halogen, $C_1$-$C_4$-Alkyl, $C_1$-$C_4$-Alkoxy, Trifluormethyl oder $C_1$-$C_4$-Alkoximino-substituiertes Aryl, gegebenenfalls durch die gleichen Reste substituiertes Heteroaryl steht oder für gegebenenfalls durch $C_1$-$C_4$-Alkyl substituiertes $C_3$-$C_8$-Cycloalkyl steht oder für geradkettiges oder verzweigtes, gegebenenfalls ein- oder mehrfach durch Halogen, $C_3$-$C_6$-Cycloalkyl, Aryl, Aryloxy substituiertes $C_1$-$C_6$-Alkyl, gegebenenfalls durch die gleichen Reste substituiertes $C_2$-$C_6$-Alkenyl oder gegebenenfalls durch die gleichen Reste substituiertes $C_2$-$C_6$-Alkinyl steht, und

X für $CH_2$, O oder S steht und

n 0 oder 1 bedeutet, sowie deren pflanzenverträglichen Säureadditions-Salze oder Metallkomplexe.

**2.** Verfahren zur Herstellung der Azolverbindungen der Formel I gemäß Anspruch 1,

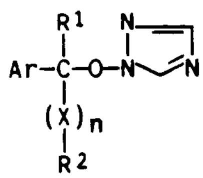

I

dadurch gekennzeichnet, daß man Verbindungen der Formel II

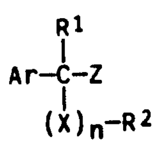

II

in welcher Ar, $R^1$, $R^2$, X und n die in Anspruch 1 oben angegebene Bedeutung haben und Z für eine nucleofuge Abgangsgruppe steht,

a) mit einem Alkalisalz der Formel III

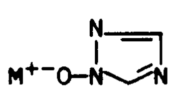

III

in der M für Natrium oder Kalium steht, ggf. in Gegenwart eines Verdünnungsmittels umsetzt

oder

b) mit 1-Hydroxy-1,2,4-triazol der Formel IV

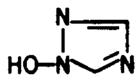

IV

in Gegenwart einer Base der Formel V $M^+B^-$ in welcher M für Natrium oder Kalium steht und B die Bedeutung Hydroxid, Alkoxid, Carbonat oder Hydrid hat, ggf. in Gegenwart eines Verdünnungsmittels umsetzt und die so erhaltenen N-Hydroxytriazolderivate ggf. mit Säure in ihre pflanzenverträglichen Salze oder ggf. mit Metallsalzen in ihre pflanzenverträglichen Metallkomplexe überführt.

**3.** Fungizid, enthaltend einen festen oder flüssigen Trägerstoff und eine fungizid wirksame Menge einer Verbindung der allgemeinen Formel I gemäß Anspruch 1.

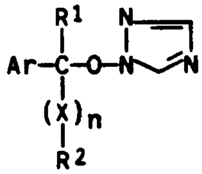

I

**4.** Verfahren zur Bekämpfung von Pilzen, dadurch gekennzeichnet, daß man eine fungizid wirksame Menge einer Verbindung der allgemeinen Formel I gemäß Anspruch 1

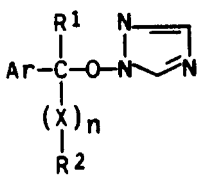

I

oder deren pflanzenverträgliches Säureadditions-Salz oder Metallkomplex auf die Pilze oder auf durch Pilzbefall bedrohte Materialien, Pflanzen, Saatgüter oder den Boden einwirken läßt.

**5.** Mittel zur Regulierung des Pflanzenwachstums, enthaltend einen festen oder flüssigen Trägerstoff und eine pflanzenwuchsregulierend wirksame Menge einer Verbindung der allgemeinen Formel I gemäß Anspruch 1.

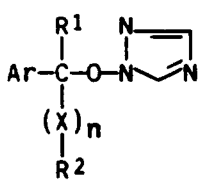

I

**6.** Verfahren zur Regulierung des Pflanzenwachstums, dadurch gekennzeichnet, daß man eine pflanzenwuchsregulierende Menge einer Verbindung der allgemeinen Formel I gemäß Anspruch 1

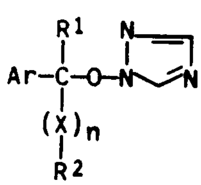

I

-oder deren pflanzenverträgliches Säureadditions-Salz oder Metallkomplex auf die Pflanzen, deren Samen oder den Boden einwirken läßt.

**7.** Verbindung der Formel I gemäß Anspruch 1, dadurch gekennzeichnet, daß Ar 4-Fluorphenyl, $R^1$ H, n = O und $R^2$ 4-Fluorphenyl bedeutet.

**8.** Verbindung der Formel I gemäß Anspruch 1, dadurch gekennzeichnet, daß Ar 2,4-Dichlorphenyl, $R^1$ H, n = O und $R^2$ n-Propyl bedeutet.

## Claims

**1.** A compound of the formula I

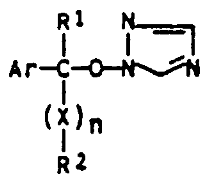

I

where

- Ar is phenyl which is unsubstituted or monosubstituted or polysubstituted by halogen, $C_1$-$C_4$-alkoxy, $C_1$-$C_4$-alkyl, trifluoromethyl, $C_1$-$C_4$-alkoximino or by unsubstituted or halogen- or trifluoromethyl-substituted phenyl or phenoxy, or is pyridyl which is unsubstituted or substituted by the same radicals, thienyl which is unsubstituted or substituted by the same radicals or naphthyl which is unsubstituted or substituted by the same radicals,
- $R^1$ is hydrogen or, where n is 0, is additionally CN,
- $R_2$ is aryl which is unsubstituted or monosubstituted or polysubstituted by halogen, $C_1$-$C_4$-alkyl, $C_1$-$C_4$-alkoxy, trifluoromethyl or $C_1$-$C_4$-alkoximino, or is hetaryl which is unsubstituted or substituted by the same radicals, or is unsubstituted or $C_1$-$C_4$-alkyl-substituted $C_3$-$C_8$-cycloalkyl or is straight-chain or branched $C_1$-$C_6$-alkyl which is unsubstituted or monosubstituted or polysubstituted by halogen, $C_3$-$C_6$-cycloalkyl, aryl or aryloxy, or is $C_2$-$C_6$-alkenyl which is unsubstituted or substituted by the same radicals, or is $C_2$-$C_6$-alkynyl which is unsubstituted or substituted by the same radicals,
- X is $CH_2$, O or S and
- n is 0 or 1,

or a plant-tolerated acid addition salt or metal complex thereof.

**2.** A process for the preparation of an azole compound of the formula I

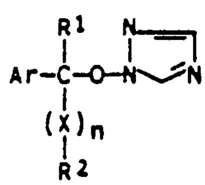

I

as claimed in claim 1, wherein a compound of the formula II

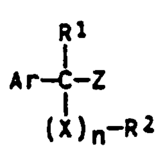

II

where
Ar, $R^1$, $R^2$, X and n have the meanings stated above in claim 1 and Z is a nucleofugic leaving group, is reacted

a) with an alkali metal salt of the formula III

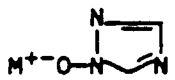

III

where M is sodium or potassium, in the presence or absence of a diluent,
or

b) with 1-hydroxy-1,2,4-triazole of the formula IV

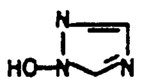

IV

in the presence of a base of the formula V $M^+ B^-$ where M is sodium or potassium and B is hydroxide, alkoxide, carbonate or hydride, in the presence or absence of a diluent, and the resulting N-hydroxytriazole derivative is, if required, converted with an acid into its plant-tolerated salts or, if necessary, with a metal salt into its plant-tolerated metal complexes.

**3.** A fungicide containing a solid or liquid carrier and a fungicidally effective amount of a compound of the general formula I

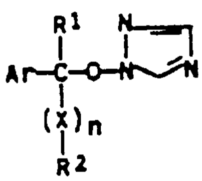

I

as claimed in claim 1.

**4.** A method of controlling fungi, wherein a fungicidally effective amount of a compound of the general formula I

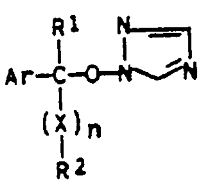

I

as claimed in claim 1, or a plant-tolerated acid addition salt or metal complex thereof, is allowed to act on the fungi, or the materials, plants, seed or soil threatened by fungal attack.

**5.** A plant growth regulator containing a solid or liquid carrier and a growth-regulating amount of a compound of the general formula I

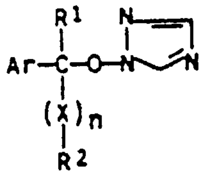

I

as claimed in claim 1.

**6.** A method for regulating plant growth, wherein a growth-regulating amount of a compound of the general formula I

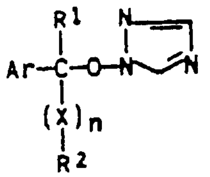

I

as claimed in claim 1, or a plant-tolerated acid addition salt or metal complex thereof, is allowed to act on the plants, their seed or the soil.

**7.** A compound of the formula I as claimed in claim 1, wherein Ar is 4-fluorophenyl, $R^1$ is H, n is 0 and $R^2$ is 4-fluorophenyl.

**8.** A compound of the formula I as claimed in claim 1, wherein Ar is 2,4-dichlorophenyl, $R^1$ is H, n is 0 and $R^2$ is n-propyl.

**Revendications**

**1.** Composés de formule I

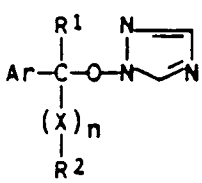

I

dans laquelle
Ar représente un groupe phényle portant éventuellement un ou plusieurs substituants halogéno, alcoxy en $C_1$-$C_4$, alkyle en $C_1$-$C_4$, trifluorométhyle, alcoximino en $C_1$-$C_4$, phényle ou phénoxy éventuellement substitué par des halogènes ou des groupes trifluorométhyle, un groupe pyridyle portant éventuellement les mêmes substituants, un groupe thiényle portant éventuellement les mêmes substituants ou un groupe naphtyle portant éventuellement les mêmes substituants,
$R^1$ représente l'hydrogène ou bien encore, lorsque n = 0, le groupe CN,
$R^2$ représente un groupe aryle portant éventuellement un ou plusieurs substituants halogéno, alkyle en $C_1$-$C_4$, alcoxy en $C_1$-$C_4$, trifluorométhyle ou alcoximino en $C_1$-$C_4$, un groupe hétéroaryle portant éventuellement les mêmes substituants ou un groupe cycloalkyle en $C_3$-$C_8$ éventuellement substitué par un groupe alkyle en $C_1$-$C_4$ ou un groupe alkyle en $C_1$-$C_6$ à chaîne droite et/ou ramifiée portant éventuellement un ou plusieurs substituants halogéno, cycloalkyle en $C_3$-$C_6$, aryle, aryloxy, un groupe alcényle en $C_2$-$C_6$ portant éventuellement les mêmes substituants ou un groupe alcynyle en $C_2$-$C_6$ portant éventuellement les mêmes substituants, et
X représente $CH_2$, O ou S et
n est égal à O ou 1,

et leurs sels formés par addition avec des acides et complexes métalliques tolérés par les vététaux.

**2.** Procédé de préparation des dérivés azoliques de formule I de la revendication 1

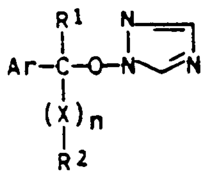

I

caractérisé en ce que l'on fait réagir des composés de formule II

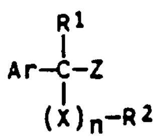

II

dans laquelle Ar, $R^1$, $R^2$, X et n ont les significations indiquées dans la revendication 1 et Z représente un groupe éliminable nucléofuge,
avec un sel alcalin de formule III

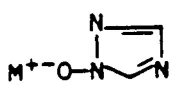

III

dans laquelle M représente le sodium ou le potassium, éventuellement en présence d'un diluant,
ou bien
avec le 1-hydroxy-1,2,4-triazole de formule IV

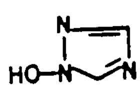

IV

en présence d'une base de formule $VM^+B^-$ dans laquelle M représente le sodium ou le potassium et B représente un radical hydroxyde, alcoolate, carbonate ou hydrure, éventuellement en présence d'un diluant, après quoi, le cas échéant, on convertit les dérivés de N-hydroxytriazole ainsi obtenus, à l'aide d'acides, en sels tolérés par les végétaux ou, à l'aide de sels métalliques, en complexes métalliques tolérés par les végétaux.

**3.** Produit fongicide contenant un véhicule solide ou liquide et une quantité fongicide efficace d'un composé de formule générale I selon la revendication 1

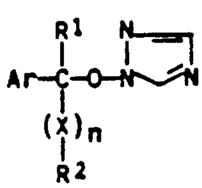

I

4. Procédé pour combattre les mycètes, caractérisé en ce que l'on fait agir sur les mycètes ou les matériaux, végétaux, semences ou sol menacés d'une attaque par les mycètes une quantité fongicide efficace d'un composé de formule générale I selon la revendication 1

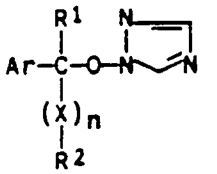

I

ou de l'un de ses sels formés par addition avec des acides ou complexes métalliques tolérés par les végétaux.

5. Produit pour la régulation de la croissance des végétaux, contenant un véhicule solide ou liquide et une quantité régulatrice efficace d'un composé de formule générale I de la revendication 1

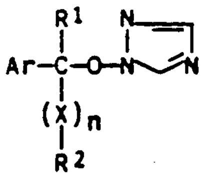

I

6. Procédé pour la régulation de la croissance des végétaux, caractérisé en ce que l'on fait agir sur les végétaux, leurs semences ou le sol une quantité régulatrice efficace d'un composé de formule générale I de la revendication 1

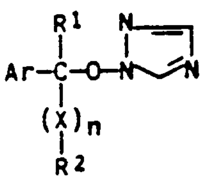

I

ou de l'un de ses sels formés par addition avec des acides ou complexes métalliques tolérés par les végétaux.

7. Composé de formule I de la revendication 1, caractérisé en ce que Ar représente un groupe 4-fluorophényle, $R^1$ représente H, n = 0 et $R^2$ représente un groupe 4-fluorophényle.

8. Composé de formule I de la revendication 1, caractérisé en ce que Ar représente un groupe 2,4-dichlorophényle, $R^1$ représente H, n = 0 et $R^2$ représente un groupe n-propyle.